(19)

Europäisches
Patentamt
European
Patent Office
Office européen
des brevets

(11) **EP 2 168 494 A1**

(12) **EUROPEAN PATENT APPLICATION**

(43) Date of publication:
**31.03.2010 Bulletin 2010/13**

(51) Int Cl.:
*A61B 8/02* (2006.01)    *A61B 8/14* (2006.01)
*G01S 7/52* (2006.01)    *G01S 15/89* (2006.01)

(21) Application number: **09170156.5**

(22) Date of filing: **14.09.2009**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**
Designated Extension States:
**AL BA RS**

(30) Priority: **26.09.2008 KR 20080094567**

(71) Applicant: **MEDISON CO., LTD.**
**Gangwon-do 250-875 (KR)**

(72) Inventors:
• **Yoo, Jae Heung**
**Seoul 135-851 (KR)**
• **Kim, Sung Yoon**
**Seoul 135-851 (KR)**

(74) Representative: **Schmid, Wolfgang**
**Lorenz & Kollegen**
**Patentanwälte Partnerschaftsgesellschaft**
**Alte Ulmer Straße 2**
**89522 Heidenheim (DE)**

(54) **Ultrasound volume data processing**

(57)    Embodiments for processing volume data in an ultrasound diagnostic system are disclosed. A volume data processing device comprises a volume data acquisition unit. The volume data acquisition acquires volume data having a plurality of frames from a periodically moving target object. Each of the frames includes a plurality of pixels. A period setting unit sets a feature point at each of the frames based on values of the pixels included therein and sets a moving period of the target object based on the feature points set at the frames. A volume data reconstructing unit reconstructs the volume data based on the set moving period.

FIG. 1

EP 2 168 494 A1

**Description**

The present application claims priority from Korean Patent Application No. 10-2008-0094567 filed on September 26, 2008, the entire subject matter of which is incorporated herein by reference.

TECHNICAL FIELD

The present disclosure generally relates to ultrasound imaging, and more particularly to ultrasound volume data processing to visualize a moving object in a 3-dimensional ultrasound image.

BACKGROUND

An ultrasound diagnostic system has become an important and popular diagnostic tool since it has a wide range of applications. Specifically, due to its non-invasive and non-destructive nature, the ultrasound diagnostic system has been extensively used in the medical profession. Modem high-performance ultrasound diagnostic systems and techniques are commonly used to produce two or three-dimensional diagnostic images of internal features of an object (e.g., human organs).

Recently, the ultrasound diagnostic system has been improved to provide a 3-dimensional ultrasound image. A static 3-dimensional ultrasound image, which is one of the 3-dimensional ultrasound images, is often used for ultrasound diagnostic purposes. By using the static 3-dimensional ultrasound image, it is possible to perform accurate observations, diagnoses or treatments of the human body without conducting complicated procedures such as invasive operations. However, the static 3-dimensional image may not be useful in certain cases, for example, in observing a moving target object in real time such as a fetus in the uterus.

To overcome this shortcoming, a live 3-dimensional imaging method and apparatus for providing a 3-dimensional moving image (rather than the static 3-dimensional image) has been developed. The live 3-dimensional image can show the movement of a moving target object more smoothly than the static 3-dimensional image.

Further, there has been an increased interest in the heart conditions of a fetus since there is an increasing need to perform an early diagnosis of the fetus' status. However, since the systole and diastole of the heart tend to rapidly repeat, it is impossible to scan all the movements of the heart just by using a 3-dimensional probe. Thus, there is a problem in providing a real heartbeat image.

SUMMARY

Embodiments for processing volume data are disclosed herein. In one embodiment, by way of non-limiting example, a volume data processing device, comprises: a volume data acquisition unit operable to acquire ultrasound volume data consisting of a plurality of image frames representing a periodically moving target object, wherein each of the frames includes a plurality of pixels; a period setting unit operable to set a feature point for each of the frames and set a moving period of the target object based on the feature points set for the image frames; and a volume data reconstructing unit operable to interpolate the ultrasound volume data to have the same number of the image frames within each moving period and reconstruct the interpolated ultrasound volume data into a plurality of sub volumes based on the moving period.

In another embodiment, a volume data processing method, comprises: a) acquiring volume data having a plurality of frames from a periodically moving target object, wherein each of the frames includes a plurality of pixels; b) setting a feature point at each of the frames based on values of the pixels included therein; c) setting a moving period of the target object based on the feature points set at the frames; d) interpolating the ultrasound volume data to have the same number of the image frames within each moving period; and e) reconstructing the interpolated ultrasound volume data into a plurality of sub volumes based on the moving period.

The Summary is provided to introduce a selection of concepts in a simplified form that are further described below in the Detailed Description. This Summary is not intended to identify key or essential features of the claimed subject matter, nor is it intended to be used in determining the scope of the claimed subject matter.

BRIEF DESCRIPTION OF THE DRAWINGS

FIG. 1    is a block diagram showing an illustrative embodiment of an ultrasound image processing device.
FIG. 2    is a block diagram showing an illustrative embodiment of a period detecting unit.
FIG 3    is a schematic diagram showing an example of setting a feature point at each of the frames.

FIG 4 is a schematic diagram showing an example of forming a feature point curve based on distances between a principle axis and feature points.

FIG. 5 is a schematic diagram showing an example of a feature point curve.

FIG. 6 is a block diagram showing an illustrative embodiment of a period setting section.

FIG. 7 is a schematic diagram showing a procedure of reconstructing volume data based on a moving period of a target object.

## DETAILED DESCRIPTION

A detailed description may be provided with reference to the accompanying drawings. One of ordinary skill in the art may realize that the following description is illustrative only and is not in any way limiting. Other embodiments of the present invention may readily suggest themselves to such skilled persons having the benefit of this disclosure.

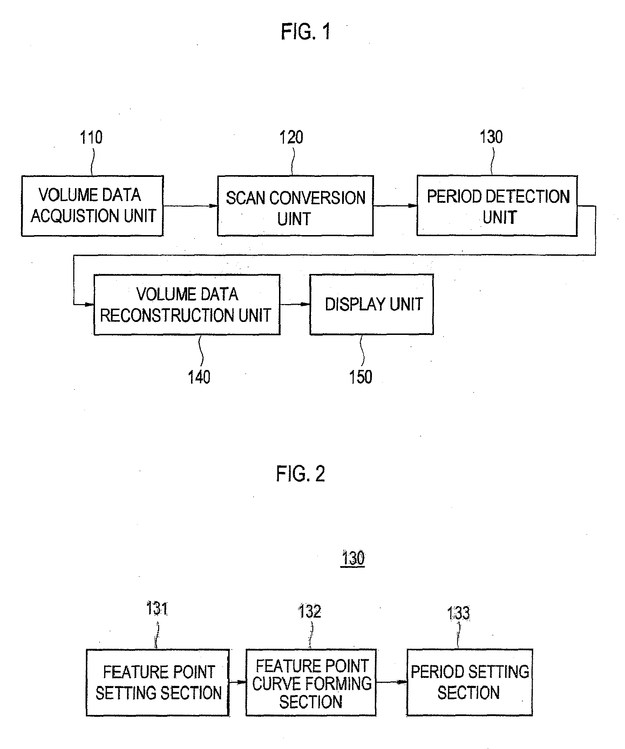

FIG. 1 is a block diagram showing an illustrative embodiment of an ultrasound image processing device. As shown in FIG. 1, the ultrasound image processing device 100 may include a volume data acquisition unit 110, a scan conversion unit 120, a period detection unit 130, a volume data reconstruction unit 140 and a display unit 150.

The volume data acquisition unit 110 may include a probe (not shown) that may be operable to transmit ultrasound signals into a target object and receive echo signals reflected from the target object. The probe may further be operable to convert the received echo signals into electrical receive signals. The volume data acquisition unit 110 may further include a beam former (not shown) that may be operable to form a receive-focused beam based on the electrical receive signals, and a signal processor (not shown) that may be operable to perform signal processing upon the receive-focused beam to thereby form a plurality of frames constituting volume data.

The scan conversion unit 120 may be coupled to the volume data acquisition unit 110 to receive the plurality of frames. The scan conversion unit 120 may be operable to perform the scan conversion upon the plurality of frames into a data format suitable for display on the display unit 150.

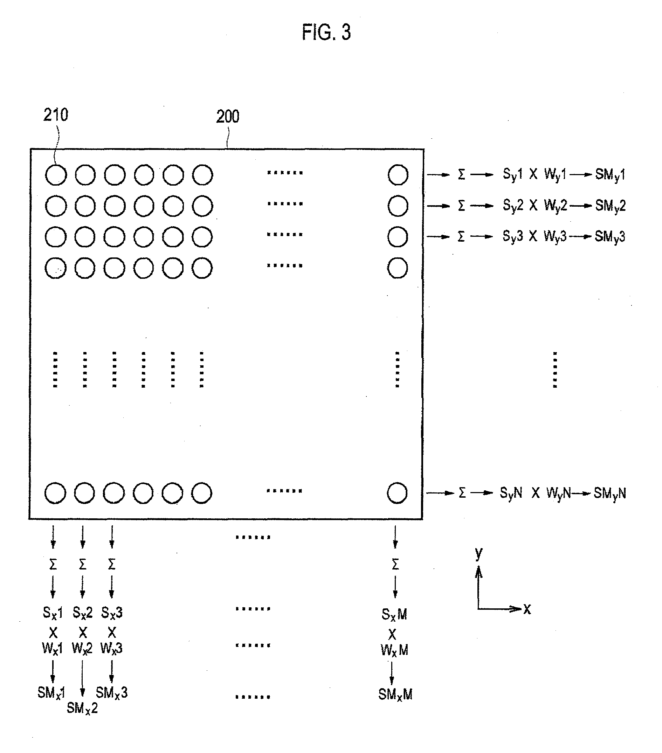

The period detection unit 130 may include a feature point setting section 131, a feature point curve forming section 132 and a period setting section 133, as illustrated in FIG. 2. The feature point setting section 131 may be operable to set a feature point at each of the frames, which are outputted from the scan conversion unit 120. The feature point may be set by using a common feature at each of the frames. In one embodiment, the feature point may be set by using a centroid of pixel values (intensities) constituting each of the frames. A method of determining a centroid of pixel values will be described by using a frame 200 having M N pixels 210, as shown in FIG. 3 as an example. For the sake of convenience, it will be described that the frames are placed on the X-Y coordinate system in which the X coordinates of the frame range from 1 to M and the Y coordinates of the frame range from 1 to N. The feature point setting section 131 may be operable to vertically sum pixel values at each of the X coordinates 1-M in the frame. That is, assuming that pixel values in the frame are represented by $P_{XY}$, the feature point setting section 130 may be operable to sum $P_{X1}$, $P_{X2}$, ... and $P_{XN}$ to thereby output first sums Sx1-SxM corresponding to respective X coordinates. Subsequently, the feature point setting section 131 may further be operable to multiply the first sums Sx1-SxM by weights Wx1-WxM, respectively, to thereby output first weighted sums SMx1-SMxM. In one embodiment, the weights Wx1-WxM may be determined by arbitrary values, which increase or decrease at a constant interval. For example, the numbers 1-M may be used as the weight values Wx1-WxM. The feature point setting section 131 may further be operable to sum all of the first sums Sx1-SxM to thereby output a second sum, and sum all of the first weighted sums SMx1-SMxM to thereby output a third sum. The feature point setting section 131 may further be operable to divide the third sum by the second sum, and then set the division result as the centroid on the X axis.

Also, the feature point setting section 131 may be operable to horizontally sum pixel values at each of the Y coordinates 1-N in the frame. That is, assuming that pixel values in the frame are represented by $P_{XY}$, the feature point setting section 130 may be operable to sum $P_{1Y}$, $P_{2Y}$, ... and $P_{MY}$ to thereby output fourth sums Sy1-SyN corresponding to respective Y coordinates. Subsequently, the feature point setting section 131 may further be operable to multiply the fourth sums Sy1-SyN by weights Wy1-WyN, respectively, to thereby output second weighted sums SMy1-SMyN. In one embodiment, the weights Wy1-WyN may be determined by arbitrary values, which increase or decrease at a constant interval. For example, the numbers 1-N may be used as the weight values Wy1-WyN. The feature point setting section 131 may further be operable to sum all of the fourth sums Sy1-SyN to thereby output a fifth sum, and sum all of the second weighted sums SMy1-SMyN to thereby output a sixth sum. The feature point setting section 131 may further be operable to divide the sixth sum by the fifth sum, and then set the division result as the centroid on the Y axis.

Although it is described that the feature point is set by using the centroid of pixel values (intensities) constituting each of the frames, the feature point setting is certainly not limited thereto. The feature point at each of the frames may be set through singular value decomposition upon each of the frames.

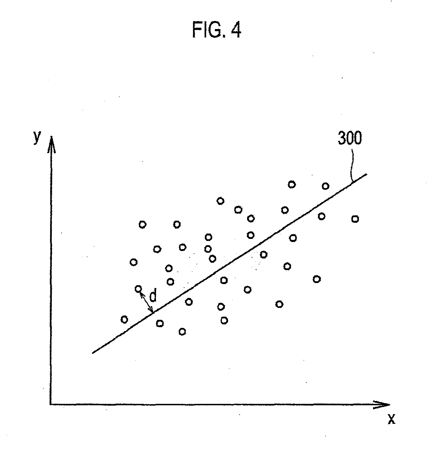

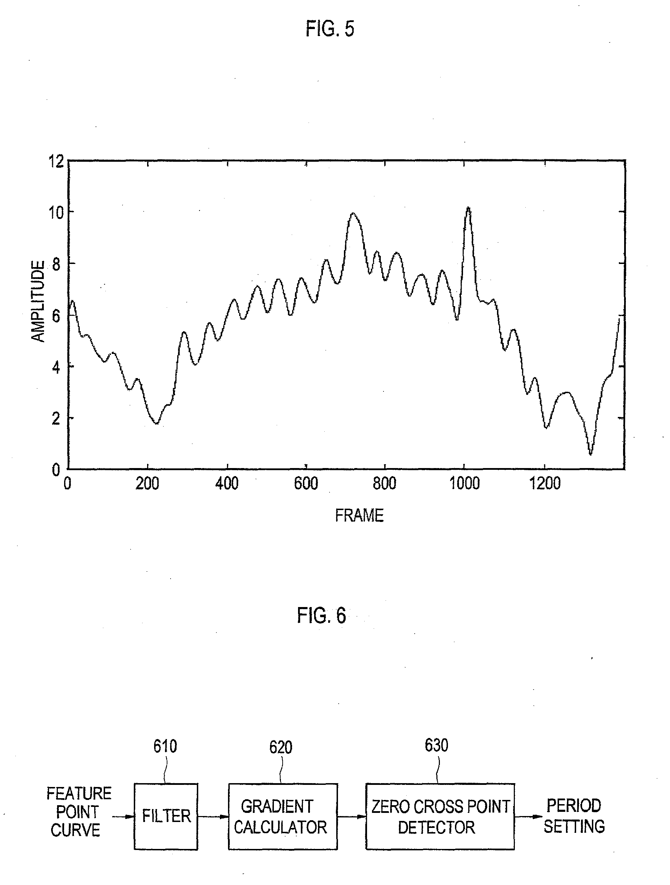

Once the setting of the centroid is complete for all of the frames, the feature point curve forming section 132 may be operable to display centroids on the X-Y coordinate system, and then set a principle axis 300 thereon, as illustrated in FIG. 4. The feature point curve forming section 132 may further be operable to compute a distance "d" from the principle axis 300 to each of the centroids. The feature point curve forming section 132 may further be operable to form a curve by using the computed distances, as illustrated in FIG 5. In FIG 5, the horizontal axis represents a frame and the vertical axis represents magnitude associated with the distances. The period setting section 133 may be operable to set a moving period of the target object by using peak points in the graph illustrated in FIG. 5, as will be explained below.

FIG. 6 is a block diagram showing a procedure of detecting the moving period in the period setting section 133. The period setting section 133 may include a filter 610, a gradient calculator 620 and a zero cross point detector 630. The filter 610 may be operable to perform filtering upon the feature point curve to reduce noises included therein. In one embodiment, a low pass filter may be used as the filter 610.

However, the filter may not be limited thereto. The filter 610 may be operable to perform Fourier transformation upon the feature point curve and search for frequencies of high amplitude. Thereafter, the filter 610 may further be operable to set a predetermined size of window such that the window contains the searched frequencies, and then perform low pass filtering upon frequencies within the window to thereby remove the noises. The filter 610 may further be operable to perform inverse Fourier transformation to thereby feature point curve with the noises removed. The gradient calculator 620 may be operable to calculate the gradients in the filtered curve. The zero cross point detector 630 may be operable to calculate zero cross points, the gradient of which is changed from positive to negative, and then detects the zero cross points having a similar distance, thereby setting a period of the detected zero cross points to the moving period of the target object.

In one embodiment, the period detection unit 130 may further include a region of interest (ROI) setting section (not shown) that may be operable to set a region of interest in each of the image frames for calculation reduction. The ROI setting section may be operable to perform horizontal projection for obtaining a projected value summing the brightness of all pixels along a horizontal pixel line in the image frame. Boundaries $n_T$ and $n_B$ of ROI can be calculated by using equation (1) shown below.

$$n_T = \min_n \{n \mid f_n < Mean\}, \quad 0 \leq n < \frac{N}{2}$$
$$n_B = \max_n \{n \mid f_n < Mean\}, \quad \frac{N}{2} \leq n < N \tag{1}$$

wherein, $f_n$ represents a horizontally projected signal, *Mean* represents a mean of the projected values, $n_T$ represents a vertical position of a projected value (in the most left side among the projected values smaller than a mean value), and $n_B$ represents a vertical position of a projected value (in the most right side among the projected values smaller than a mean value). $n_T$ and $n_B$ are used as the boundaries of ROI. The ROI setting section may further be operable to mask the image frame by using the boundaries $n_T$ and $n_B$ of ROI, thereby removing regions that are located outside the boundaries $n_T$ and $n_B$ from the image.

The volume data reconstructing unit 160 may be operable to perform interpolation upon the volume data to have the same number of the frames within each period.

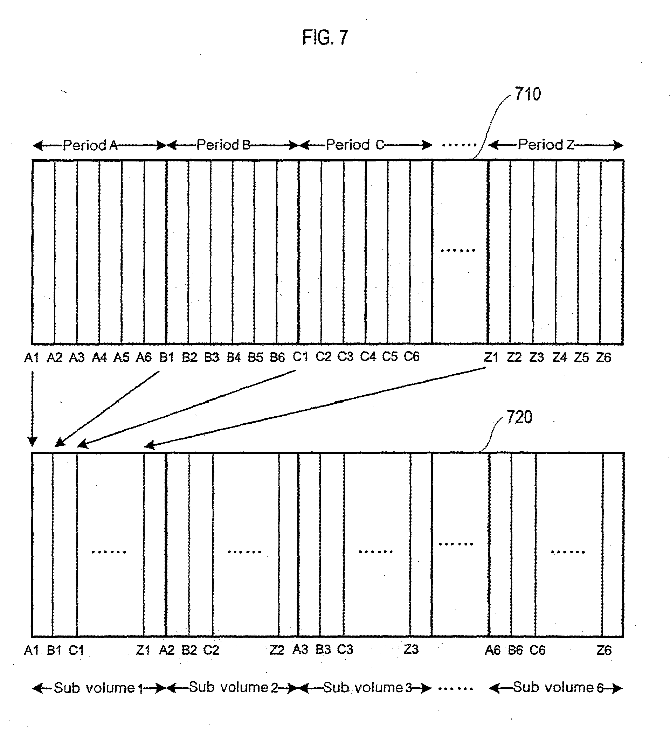

After completing the interpolation, the volume data reconstructing unit 140 reconstructs the interpolated volume data to provide a 3-dimensional ultrasound image showing a figure of the heartbeat in accordance with the present invention. FIG 7 shows a procedure of reconstructing the interpolated volume data. As shown in FIG. 9, twenty-six local periods A to Z exist in one volume data 710. Assuming that six frames are contained in one period in the volume data as shown in FIG. 7, the reconstructed volume data 720 may include six sub volumes. Each of the sub volumes may consist of 26 frames $A_i$ to $Z_i$.

Further, when the 3-dimensional volume data are acquired by scanning the target object, the object (e.g., expectant mother or fetus) may be moved. This makes it difficult to accurately detect the heartbeat of the fetus. Accordingly, the ultrasound image processing device may further include a motion compensating unit (not shown). The motion compensating unit may be operable to compensate the motion of the expectant mother or the fetus by matching the brightness of pixels between a previously set VOI and a currently set VOI. The motion compensating unit calculates the motion vectors by summing the absolute differences of brightness of pixels between the previously set VOI and the currently set VOI. For example, assuming that VOI at a nth frame is expressed as $V^n(m)$, VOI at a next frame can be expressed as $V^n(m+1)$. In such a case, a variable m represents the combination of n-1, n and n+1. The motion compensating unit moves $V^n(m)$ up, down, right and left (i, j), and then calculates the absolute differences of brightness of pixels between $V^n(m)$ and $V^n(m+1)$ at each position. A motion vector is estimated at a position where the absolute difference is minimal. The sum of the absolute difference is calculated as the following equation (2).

$$SAD_n(i,j) = \sum_{m=-1}^{1}\sum_{l=0}^{M-1}\sum_{k=n_T}^{n_B} |V^n(m,k,l) - V_{i,j}^n(m+1,k,l)|$$

(2)

$$for \quad -W \le i, j < W, \quad 1 \le n < K-1$$

wherein, $W$ represents a predefined motion estimated range, $K$ represents a total number of the frames, $i,j$ represent motion displacements, $k,l$ represent the position of a pixel in the frame included in VOI, and m represents the number of the frames.

Since the volume data are reconstructed in accordance with the moving period, an improved ultrasound image of the target object can be provided. Also, since the motion of the expectant mother or the fetus is compensated, the ultrasound image can be more accurately and clearly provided.

Although embodiments have been described with reference to a number of illustrative embodiments thereof, it should be understood that numerous other modifications and embodiments can be devised by those skilled in the art that will fall within the spirit and scope of the principles of this disclosure. More particularly, numerous variations and modifications are possible in the component parts and/or arrangements of the subject combination arrangement within the scope of the disclosure, the drawings and the appended claims. In addition to variations and modifications in the component parts and/or arrangements, alternative uses will also be apparent to those skilled in the art.

**Claims**

1. An ultrasound volume data processing device, comprising:

   a volume data acquisition unit configured to acquire ultrasound volume data consisting of a plurality of image frames representing a periodically moving target object, wherein each of the frames includes a plurality of pixels;
   a period setting unit configured to set a feature point for each of the frames and set a moving period of the target object based on the feature points set for the image frames; and
   a volume data reconstructing unit configured to interpolate the ultrasound volume data to have the same number of the image frames within each moving period and reconstruct the interpolated ultrasound volume data into a plurality of sub volumes based on the moving period.

2. The volume data processing device of Claim 1, wherein the period setting unit includes:

   a feature point setting section configured to set the feature point at each of the frames based on values of the pixels included therein;
   a feature point curve forming section configured to form a feature point curve based on the feature points; and
   a period setting section configured to set the moving period of the target object based on the feature points set at the frames.

3. The volume data processing device of Claim 2, wherein the feature point setting section is configured to set a centroid of the pixel values at each of the frames to the feature point.

4. The volume data processing device of Claim 2, wherein the feature point curve forming section is configured to set a principle axis based on positions of the feature points and form the feature point curve based on distances between the feature points and the principle axis.

5. The volume data processing device of Claim 4, wherein the period setting section includes:

   a filter configured to perform filtering upon the feature point curve to reduce noises;
   a gradient calculator configured to calculate gradients from the filtered feature point curve; and
   a zero crossing point detector configured to detect zero crossing points changing a sign of the gradient from positive to negative and determine the moving period based on intervals between the detected zero crossing points.

6. The volume data processing device of Claim 1, further comprising a motion compensation unit configured to estimate a motion of the target object in the volume data to compensate for the motion.

7. A method of processing volume date, comprising:

    a) acquiring volume data having a plurality of frames from a periodically moving target object, wherein each of the frames includes a plurality of pixels;
    b) setting a feature point at each of the frames based on values of the pixels included therein;
    c) setting a moving period of the target object based on the feature points set at the frames;
    d) interpolating the ultrasound volume data to have the same number of the image frames within each moving period; and
    e) reconstructing the interpolated ultrasound volume data into a plurality of sub volumes based on the moving period.

8. The method of Claim 7, wherein the step c) includes:

    c1) forming a feature point curve based on the feature points; and
    c2) setting the moving period of the target object based on the feature points set at the frames.

9. The method of Claim 8, wherein a centroid of the pixel values at each of the frames is set to the feature point.

10. The method of Claim 8, wherein the step c1) includes:

    setting a principle axis based on positions of the feature points; and
    forming the feature point curve based on distances between the feature points and the principle axis.

11. The method of Claim 10, wherein the step c2) includes:

    performing filtering upon the feature point curve to reduce noises;
    calculating gradients from the filtered feature point curve; and
    detecting zero crossing points changing a sign of the gradient from positive to negative and determining the moving period based on intervals between the detected zero crossing points.

12. The method of Claim 7, further comprising estimating a motion of the target object in the volume data to compensate for the motion.

# FIG. 1

```
    110                      120                      130

┌─────────────────┐    ┌─────────────────┐    ┌─────────────────┐
│  VOLUME DATA    │    │ SCAN CONVERSION │    │ PERIOD DETECTION│
│ ACQUISTION UNIT │───▶│      UINT       │───▶│      UNIT       │──┐
└─────────────────┘    └─────────────────┘    └─────────────────┘  │
                                                                    │
  ┌─────────────────────────────────────────────────────────────────┘
  │
  │    ┌─────────────────┐    ┌─────────────────┐
  └───▶│  VOLUME DATA    │───▶│   DISPLAY UNIT  │
       │RECONSTRUCTION UNIT│    │                 │
       └─────────────────┘    └─────────────────┘

              140                      150
```

# FIG. 2

130

```
     131                    132                    133

┌─────────────────┐    ┌─────────────────┐    ┌─────────────────┐
│  FEATURE POINT  │    │  FEATURE POINT  │    │ PERIOD SETTING  │
│ SETTING SECTION │───▶│ CURVE FORMING   │───▶│    SECTION      │
│                 │    │    SECTION      │    │                 │
└─────────────────┘    └─────────────────┘    └─────────────────┘
```

7

## FIG. 3

$\Sigma \longrightarrow S_y1 \ X \ W_y1 \longrightarrow SM_y1$

$\Sigma \longrightarrow S_y2 \ X \ W_y2 \longrightarrow SM_y2$

$\Sigma \longrightarrow S_y3 \ X \ W_y3 \longrightarrow SM_y3$

$\Sigma \longrightarrow S_yN \ X \ W_yN \longrightarrow SM_yN$

$\Sigma \quad \Sigma \quad \Sigma \qquad\qquad \Sigma$

$S_x1 \quad S_x2 \quad S_x3 \qquad S_xM$
$X \quad X \quad X \qquad X$
$W_x1 \quad W_x2 \quad W_x3 \qquad W_xM$

$SM_x1 \quad SM_x3 \qquad SM_xM$
$SM_x2$

FIG. 4

## FIG. 5

## FIG. 6

FIG. 7

Europäisches
Patentamt
European
Patent Office
Office européen
des brevets

# EUROPEAN SEARCH REPORT

**Application Number**

EP 09 17 0156

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | KARADAYI K ET AL: "Automatic Image-based Gating for 4D Ultrasound" ENGINEERING IN MEDICINE AND BIOLOGY SOCIETY, 2006. EMBS '06. 28TH ANNUAL INTERNATIONAL CONFERENCE OF THE IEEE, IEEE, PISCATAWAY, NJ, USA, 30 August 2006 (2006-08-30), pages 2388-2391, XP031339478 ISBN: 978-1-4244-0032-4 * paragraphs II and III, in particular: * * sentences 1-5, paragraph II.B * * sentences 5-7, paragraph II.C * * sentences 9-10, paragraph II.C * * sentences 18-22, paragraph II.C * ----- | 1-12 | INV. A61B8/02 A61B8/14 G01S7/52 G01S15/89 |
| A | KRYSTEK, MICHAEL: "Ausgleichsgeraden in der Ebene" TM - TECHNISCHES MESSEN vol. 71, no. 1, January 2004 (2004-01), pages 19-23, XP002557991 ISSN: 0171-8096 DOI: 10.1524/teme.71.1.19.25416 Retrieved from the Internet: URL:http://www.atypon-link.com/OLD/doi/pdf /10.1524/teme.71.1.19.25416> [retrieved on 2009-12-11] * the whole document * ----- | 4,10 | TECHNICAL FIELDS SEARCHED (IPC) A61B G01S |

The present search report has been drawn up for all claims

| Place of search | Date of completion of the search | Examiner |
|---|---|---|
| The Hague | 1 December 2009 | Albrecht, Ronald |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020080094567